# FASCICULE DE BREVET EUROPEEN

(11) **EP 0 712 302 B1**
(45) Date de publication et mention de la délivrance du brevet: **28.05.1997**
(21) Numéro de dépôt: 94922301.0
(22) Date de dépôt: 22.07.1994
(51) Int. Cl.: A61F 2/24

(54) **ANNEAU PROTHETIQUE POUR CHIRURGIE CARDIAQUE**
RINGPROTHESE FÜR HERZCHIRURGIE
PROSTHETIC RING FOR USE IN CARDIAC SURGERY

(30) Priorité: 03.08.1993 FR 9309768
(43) Date de publication de la demande: 22.05.1996
(73) Titulaire: Seguin, Jacques, F-34000 Montpellier (FR)
(72) Inventeur: SEGUIN, Jacques, F-34000 Montpellier (FR); ROGIER, Robert, F-34200 Sète (FR)
(74) Mandataire: Maureau, Philippe
(86) Numéro de dépôt international: FR9400928
(87) Numéro de publication internationale: WO9503757

(56) Documents cités:
- EP-A- 0 338 994
- GB-A- 1 264 472
- US-A- 3 656 185
- US-A- 4 489 446
- US-A- 4 917 698
- US-A- 5 061 277
- US-A- 5 104 407

## Description

La présente invention concerne un anneau prothétique pour chirurgie cardiaque, notamment pour annuloplastie mitrale, tricuspide ou aortique.

Dans les valves mitrales, tricuspides ou aortiques normales, les valvules se recouvrent au centre de l'anneau fibromusculaire qui entoure la valve et assurent ainsi l'étanchéité de la valve contre une régurgitation du sang du ventricule vers l'oreillette ou de l'aorte dans le ventricule.

Différentes affections peuvent conduire à des déformations ou des dilatations de ces anneaux, qui génèrent un défaut d'étanchéité des valvules et, par conséquent, une régurgitation du sang.

Tant que la déformation de l'anneau n'est pas trop importante, il est préférable d'avoir recours à une plastie de reconstruction de l'anneau plutôt qu'à un remplacement total de la valve.

Un anneau prothétique prévu à cet effet comprend une âme revêtue d'une gaine en matériau textile hémocompatible, cette âme devant présenter une rigidité suffisante pour permettre de réduire les déformations ou dilatations de l'anneau natif, sans perturber, autant que faire se peut, le mouvement naturel d'ouverture et de fermeture de la valve, et la gaine permettant la suture de la prothèse à la paroi cardiaque ou aortique.

Plusieurs modèles d'anneaux prothétiques ont été développés depuis une vingtaine d'années.

Les premiers implants ont été des implants annulaires ou partiellement annulaires, rigides, à l'exemple de ceux décrits dans le document US-A-3 656 185 (CARPENTIER).

La rigidité de ces anneaux permet de réduire la dilatation de l'anneau natif et de leur redonner une forme satisfaisante mais présente, en revanche, l'inconvénient de s'opposer à la flexibilité naturelle de l'anneau de la valve mitrale postérieure et de la valve septale tricuspide, ce qui peut conduire à des disfonctionnements de ces valves. En outre, ces prothèses à anneaux rigides présentent une résistance aux mouvements naturels de la paroi cardiaque, de telle sorte que leurs sutures à la paroi cardiaque sont sollicitées et, par conséquent, soumises à usure et à distension.

Le brevet Britannique GB-1264472 décrit également un anneau de ce type, comprenant une âme monolithique en titane recouverte d'une gaine en matière textile connue sous la marque "Dacron". Cette âme présente une section aplatie, qui est d'épaisseur sensiblement constante le long de la périphérie de l'anneau et de hauteur relativement importante, de sorte que l'anneau ne présente aucune flexibilité sur sa circonférence.

A l'inverse, certains concepteurs ont proposé des anneaux prothétiques extrêmement flexibles, comme par exemple ceux décrits dans le document US-A-4 290 151 (MASSANA) et dans les travaux de DURAN publiés dans la revue "The Annals of Thoracic Surgery, Volume 22, No. 5, 458-463, (1976)". Le document US-A-4 042 979 (ANGELL) propose également un anneau souple, qui est ajustable lors de l'intervention chirurgicale à la géométrie de l'anneau considéré.

Tous ces anneaux très souples, qui autorisent trop de déformations de l'anneau fibromusculaire, ne parviennent pas toujours à restaurer une coaptation satisfaisante des valvules.

Le document US-A-4 489 446 (REED) décrit un anneau constitué par deux éléments rigides emboîtés, qui coulissent l'un dans l'autre à chaque contraction cardiaque de manière à autoriser une déformation de l'anneau dans son plan. Cet anneau présente également l'inconvénient d'être d'une trop grande rigidité, en s'opposant aux mouvements cardiaques exercés perpendiculairement au plan de l'anneau.

Plus récemment, il a été proposé des anneaux prothétiques présentant, sur leur circonférence, des portions de rigidités différentes.

Ainsi, le document US-A-4 917 698 (CARPENTIER) décrit un anneau prothétique constitué par l'assemblage de deux segments pour une valve mitrale et de trois segments pour une valve tricuspide, les segments étant liés les uns aux autres et articulés les uns par rapport aux autres au moyen d'un lien flexible en matière textile qui les traverse. L'un des segments de cet anneau est rigide et est réalisé en alliage de titane, tandis que le ou les autres segments sont flexibles et constitués en matière synthétique connue sous la marque "DELRIN". Plusieurs segments souples de dimensions différentes peuvent être assemblés à un segment rigide pour constituer un anneau, de manière à permettre de moduler la flexibilité de la partie souple de l'anneau en fonction des besoins.

Le document US-A-5 061 277 (CARPENTIER et LANE) décrit un autre anneau présentant une partie rigide en alliage de titane, constituée par un élément en forme de "C", et une partie flexible, constituée par un cordon souple relié aux extrémités dudit élément et par des segments cylindriques en matériau compressible, engagés sur le cordon et lui donnant une forme arquée. Cette liaison présente l'inconvénient de pouvoir être sujette à usure et d'affecter ainsi la résistance dans le temps de l'implant. De plus, cet anneau présente une structure relativement complexe, le rendant relativement difficile à fabriquer.

Le brevet US-A-5 104 407 (LAM, NGUYEN et CARPENTIER) décrit un anneau dont la partie interne est constituée par un enroulement en spirale de fibres fines d'un alliage de cobalt-nickel, connu sous le nom d' "ELGILOY". Les différentes strates de fibres se recouvrent dans la partie rigide, alors qu'elles sont séparées les unes des autres par un matériau élastomère dans la partie plus flexible. Le nombre de couches de fibres varie en fonction de l'élasticité à obtenir.

Cette structure permet d'obtenir une meilleure répartition sur la périphérie de l'anneau des contraintes exercées par le muscle cardiaque. Toutefois, de par sa structure, cet anneau apparaît difficilement déformable autrement que dans son plan alors que l'anneau natif est également soumis à des déformations perpendiculairement à son plan. Cette rigidité dans cette direction interfère également avec certaines composantes du mouvement cardiaque naturel. De plus, cet anneau apparaît complexe et difficile à fabriquer.

Ainsi, l'état de la technique montre que la recherche d'une flexibilité adéquate de l'anneau conduit à des structures de plus en plus complexes à fabriquer et dont la résistance dans le temps peut être incertaine.

La présente invention vise à remédier à ces inconvénients en fournissant un anneau prothétique apte à s'adapter parfaitement à l'anneau natif, sans interférer avec le mouvement cardiaque naturel, et tout en restant simple à fabriquer.

Cet anneau est du type comprenant une âme engagée dans une gaine textile constituant un moyen de suture, cette âme présentant au moins une portion relativement rigide et une portion relativement flexible par rapport à celle-ci.

Selon l'invention, l'âme forme un anneau complet, est à structure monolithique, c'est-à-dire réalisée en une seule pièce, et la section transversale de l'âme varie au long de la circonférence de l'anneau, c'est-à-dire diminue en direction de sa portion devant être plus flexible, de manière à permettre une déformation de l'anneau dans tous les plans.

L'invention s'écarte de l'évolution de la technique antérieure, pour fournir un anneau dont la flexibilité requise est obtenue de manière beaucoup plus simple que dans les anneaux antérieurs.

Le compromis entre la rigidité et la flexibilité aux endroits adéquats de cet anneau s'avère donner toute satisfaction en pratique. Il présente en effet une rigidité suffisante pour réduire la dilatation de l'anneau natif et redonner à celui-ci une géométrie satisfaisante, tout en ayant, à l'endroit adéquat, la flexibilité lui permettant de ne pas contrarier le mouvement cardiaque naturel.

En outre, il est parfaitement résistant dans de temps, tant dans sa structure même qu'au niveau des sutures assurant sa fixation à la paroi cardiaque ou aortique.

Outre une facilité de fabrication accrue, une âme monolithique ne présente aucun risque d'usure ou de rupture sous l'effet des contraintes répétées qu'elle subit, comme cela peut être le cas lorsqu'elle est constituée, selon la technique antérieure, par assemblage de plusieurs segments de matériaux différents.

La section transversale de l'anneau peut diminuer selon au moins une direction transversale, notamment selon l'épaisseur et/ou la hauteur de l'âme, de manière symétrique ou asymétrique par rapport à son centre.

De préférence, la diminution de la section est progressive, de manière régulière ou non, pour l'obtention d'une évolution progressive de la flexibilité de l'âme et pour une répartition uniforme sur sa circonférence des contraintes exercées par la paroi cardiaque ou aortique.

Selon une première possibilité, l'âme est constituée par moulage d'une matière synthétique, notamment celle connue sous la marque "DELRIN".

Selon une autre possibilité, l'âme est constituée en alliage de titane connu sous la référence TA6V.

La partie flexible peut être habillée par un insert en matériau élastomère venant renforcer l'âme, pour compenser la diminution de la section de celle-ci, si nécessaire.

Si besoin est, l'anneau peut en outre inclure un élément radio-opaque permettant de le rendre visible au cours d'une radiographie.

Pour sa bonne compréhension, l'invention va être à nouveau décrite ci-dessous en référence au dessin schématique annexé représentant, à titre d'exemple non limitatif, une forme de réalisation préférée de l'anneau prothétique qu'elle concerne.

Dans ce dessin,
la figure 1 est une vue en plan d'un anneau destiné à une annuloplastie mitrale ;
la figure 2 est une vue en plan d'un anneau destiné à une annuloplastie tricuspide ;
la figure 3 est une vue de côté d'un anneau destiné à une annuloplastie mitrale, selon une variante de réalisation ;
la figure 4 est une vue en plan d'un anneau destiné à une annuloplastie mitrale, partiellement coupé dans son plan, selon une autre variante de réalisation ;
la figure 5 en est une vue en coupe selon la ligne V-V de la figure 4 ;
la figure 6 est une vue de côté d'un anneau destiné à une annuloplastie aortique ;
la figure 7 en est une vue de dessus et
les figures 8,9 et 10 en sont des vues en coupe selon respectivement les lignes VIII-VIII, IX-IX et X-X des figures 6 ou 7.

Les anneaux prothétiques 2, 3 et 10 représentés ont chacun une forme générale anatomique, sensiblement en forme de D pour l'anneau 2 destiné à la reconstruction de l'anneau natif mitral, de forme plus ou moins ovoïde pour l'anneau 3 destiné à la reconstruction de l'anneau natif tricuspide, et comprenant trois portions courbes raccordées les unes aux autres par des commissures plus ou moins perpendiculaires au plan de l'anneau, pour l'anneau 10 destiné à la reconstruction de l'anneau natif aortique.

Chacun de ces anneaux 2,3 est constitué par une âme 4 engagée dans une gaine textile hémocompatible 5, constituant un moyen de suture à la paroi cardiaque.

Chaque âme 4 présente une portion 4a relativement rigide et une portion 4b relativement flexible par rapport à la portion 4a.

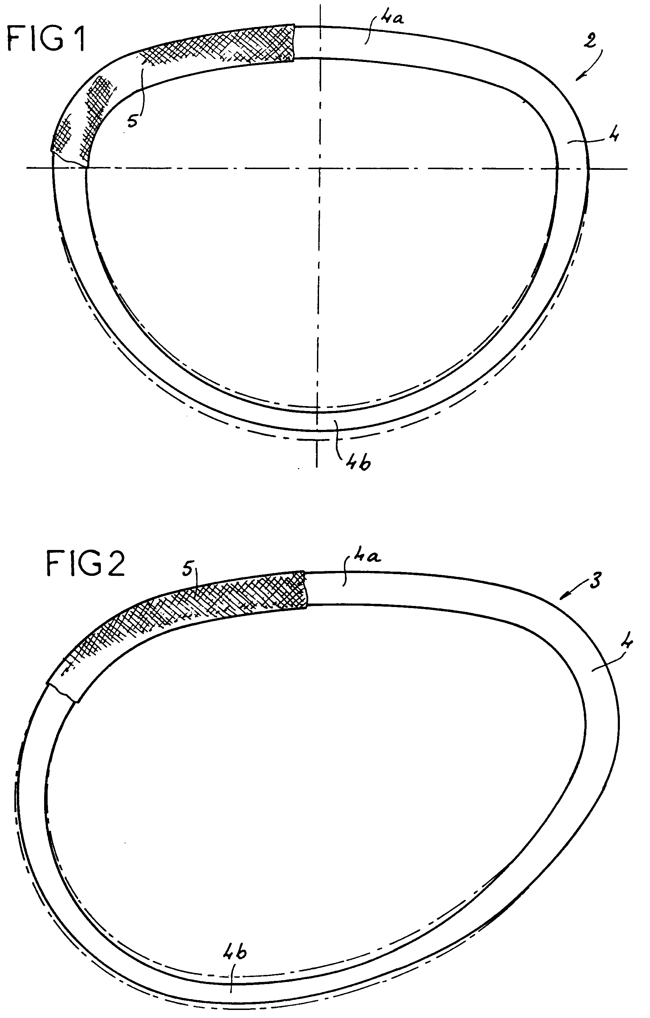

Ainsi que cela est rendu visible sur les figures 1 et 2 au moyen de traits mixtes, la section transversale de l'âme 4 varie progressivement au long de la circonférence de l'anneau 2,3, c'est-à-dire diminue progressivement à partir de sa portion la plus rigide 4a en direction de sa portion la plus flexible 4b, jusqu'à un minimum situé sensiblement à l'opposé de la partie médiane de la portion la plus rigide 4a.

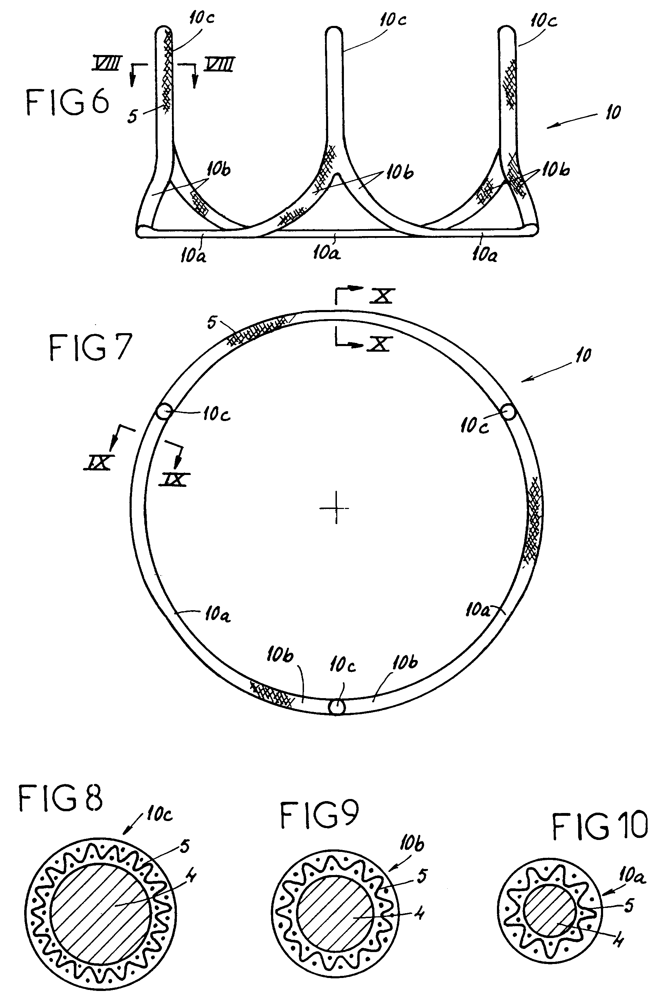

Comme cela apparaît sur la figure 5, la section transversale de l'âme 4 est circulaire et diminue tant au niveau de l'épaisseur de l'âme 4, c'est-à-dire dans le plan de l'anneau 2,3, qu'au niveau de la hauteur de l'âme 4, c'est-à-dire perpendiculairement au plan de l'anneau 2,3.

En outre, chaque âme 4 est à structure monolithique, c'est-à-dire réalisée en une seule pièce de matériau, notamment par moulage d'une matière synthétique et en particulier de celle connue sous la marque "DELRIN".

Cette diminution de section transversale permet d'obtenir la flexibilité requise de l'âme 4, à l'endroit adéquat, et ce, de manière très simple à fabriquer.

L'anneau prothétique 2,3 ainsi constitué présente une rigidité suffisante pour réduire la dilatation de l'anneau natif et redonner à celui-ci une forme satisfaisante, tout en ayant, à l'endroit adéquat, la flexibilité lui permettant de ne pas s'opposer aux mouvements naturels du coeur.

Les sutures par lesquelles l'anneau est fixé à la paroi cardiaque ne sont, dès lors, que peu sollicitées.

Grâce à la diminution précitée de la section transversale de l'âme 4 tant en épaisseur qu'en hauteur, il est obtenu une flexibilité de l'anneau 2,3 à la fois dans son plan et perpendiculairement à son plan. Cette double possibilité de déformation rend l'anneau 2,3 selon l'invention parfaitement fonctionnel.

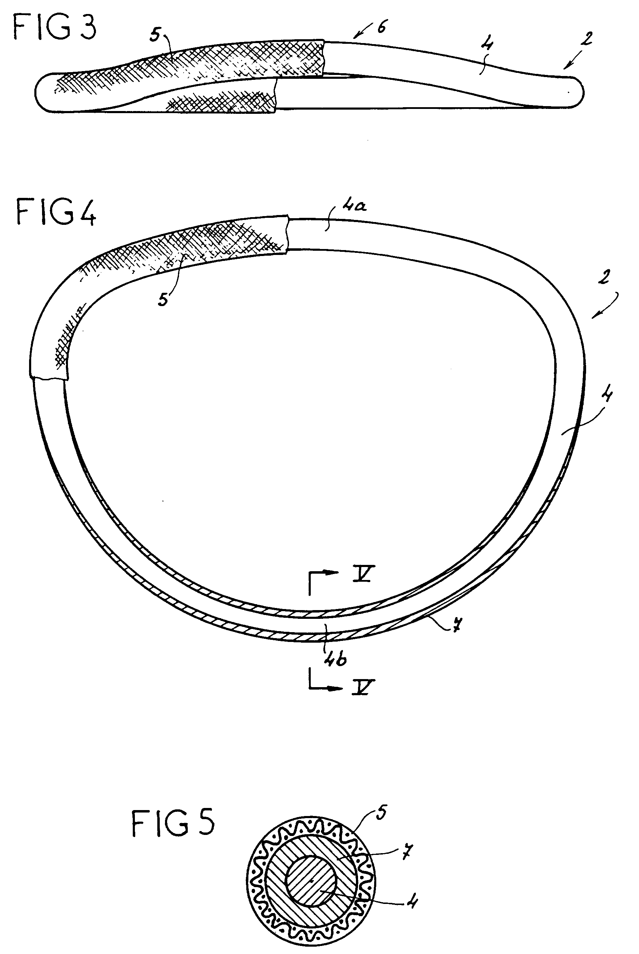

La figure 3 représente un anneau pour annuloplastie mitrale, ayant la même constitution que l'anneau 2 représenté à la figure 1 sinon qu'il présente une déformation 6 en dehors de son plan, située au niveau de la partie rigide 4a de l'âme 4 destinée à venir contre le trigone fibreux. Cette déformation 6 est symétrique par rapport à l'axe antéro-postérieur de l'anneau.

L'anneau 2, ainsi déformé, présente l'avantage d'avoir une meilleure adaptation à la géométrie naturelle de l'anneau.

La figure 4 représente une autre variante de réalisation de l'anneau mitral 2. Ici également, les éléments préalablement décrits qui se retrouvent dans cette variante sont désignés par les mêmes références.

Dans cet anneau mitral 2, l'âme 4 est constituée en alliage de titane connu sous la référence TA6V. Pour obtenir la flexibilité adéquate, la diminution de la section de l'âme 4 au niveau de sa partie 4b est importante, ainsi que le montre la figure 5. Cette diminution est alors compensée par un insert 7 en matériau élastomère biocompatible, venant renforcer l'âme 4 à cet endroit. La section intérieure de l'insert 7 évolue en complément de celle de l'âme 4, de manière à conférer à l'anneau 2 une section extérieure sensiblement constante.

Les figures 6 à 10 montrent l'anneau 10 destiné à une annuloplastie aortique.

L'anneau 10 comprend trois portions courbes 10a raccordées les unes aux autres par des commissures 10b orientées sensiblement perpendiculairement au plan de l'anneau 10, ces commissures 10b étant prolongées par des doigts 10c d'appui permettant la fixation sur la zone correspondante de l'anneau aortique et de la paroi aortique, constituant, en cela, une nouvelle technique chirurgicale.

De la même manière que précédemment décrit, cet anneau 10 est constitué par une âme 4 engagée dans une gaine textile de suture 5, l'âme 4 étant constituée par moulage d'une matière synthétique thermoplastique telle celle connue sous la marque "DELRIN".

Ainsi que le montrent les figures 7 à 10, la section transversale de l'âme 4 évolue le long de l'anneau 10, au sein de chaque portion courbe 10a. Elle est minimum au niveau de la partie médiane de chacune de ces portions courbes 10a et augmente en direction des commissures 10b.

De la même manière que pour les anneaux précédemment décrits, cet anneau 10 permet de réduire la dilatation de l'anneau aortique natif en lui rendant une forme permettant le recouvrement des valvules, tout en ayant la flexibilité lui permettant de ne pas contrarier le mouvement cardiaque. Cet anneau 10 est, ainsi, parfaitement fonctionnel.

L'invention n'est bien entendu pas limitée aux deux formes de réalisation qui viennent d'être décrites ci-dessus à titre d'exemples. Elle en embrasse au contraire toutes les variantes de réalisation.

## Revendications

1. Anneau prothétique pour annuloplastie mitrale, tricuspide ou aortique, comprenant une âme (4) engagée dans une gaine textile (5) constituant un moyen de suture, cette âme présentant au moins une portion relativement rigide (4a,10b) et une portion relativement flexible (4b,10a) par rapport à celle-ci, caractérisé en ce que l'âme (4) forme un anneau complet (2,3,10), en ce qu'elle est à structure monolithique, c'est-à-dire réalisée en une seule pièce, et en ce que la section transversale de l'âme (4) varie au long de la circonférence de l'anneau (2,3,10), c'est-à-dire diminue en direction de sa portion (4b,10a) devant être plus flexible, de manière à permettre une déformation de l'anneau (2,3,10) dans tous les plans.

2. Anneau prothétique selon la revendication 1, caractérisé en ce que la section transversale de l'âme diminue selon au moins une direction transversale, notamment selon l'épaisseur et/ou la hauteur de l'âme (4,10), de manière symétrique ou asymétrique par rapport à son centre.

3. Anneau prothétique selon la revendication 1 ou la revendication 2, caractérisé en ce que la diminution de la section transversale de l'âme (4,10) est progressive, de manière régulière ou non.

4. Anneau prothétique selon l'une des revendications 1 à 3, caractérisé en ce que l'âme (4) est constituée par moulage d'une matière synthétique.

5. Anneau prothétique selon l'une des revendications 1 à 3, caractérisé en ce que l'âme (4) est constituée en alliage de titane connu sous la référence TA6V.

6. Anneau prothétique selon l'une des revendications 4 ou 5, caractérisé en ce que la partie flexible (4b) de l'âme (4) est habillée par un insert en matériau élastomère venant la renforcer.

7. Anneau prothétique selon l'une des revendications 1 à 6, caractérisé en ce qu'il comprend un élément radio-opaque.

8. Anneau prothétique pour annuloplastie mitrale, selon l'une des revendications 1 à 7, caractérisé en ce qu'il présente une forme générale sensiblement en forme de D, en ce que la section transversale de l'âme (4) varie progressivement au long de la circonférence de l'anneau (2), à partir de sa portion la plus rigide (4a) et en direction de sa portion la plus flexible (4b), jusqu'à un minimum situé sensiblement à l'opposé de la partie médiane de la portion la plus rigide (4a).

9. Anneau prothétique pour annuloplastie tricuspide, selon l'une des revendications 1 à 7, caractérisé en ce qu'il présente une forme générale plus ou moins ovoïde, et en ce que la section transversale de l'âme (4) varie progressivement au long de la circonférence de l'anneau (2), à partir de sa portion la plus rigide (4a) et en direction de sa portion la plus flexible (4b), jusqu'à un minimum situé sensiblement à l'opposé de la partie médiane de la portion la plus rigide (4a).

10. Anneau prothétique pour annuloplastie aortique, selon l'une des revendications 1 à 7, caractérisé en ce qu'il présente trois portions courbes (10a) raccordées les unes aux autres par des commissures (10b) plus ou moins perpendiculaires au plan de l'anneau, ces commissures (10b) étant prolongées par des doigts d'appui (10c) permettant la fixation sur la zone correspondante de l'anneau aortique et de la paroi aortique, et en ce que la section transversale de l'âme évolue le long de l'anneau (10), au sein de chaque portion courbe (10a), en étant minimum au niveau de la partie médiane de chacune de ces portions courbes (10a) et en augmentant en direction des commissures (10b).

## Claims

1. Prosthetic ring for mitral, tricuspid or aortic annuloplasty, comprising a core (4) which is fitted in a textile sheath (5) constituting a suturing means, this core having at least one relatively rigid portion (4a, 10b) and a portion (4b, 10a) which is relatively flexible in relation to the latter, characterized in that the core (4) forms a complete ring (2, 3, 10), is of monobloc structure, that is to say is made in a single piece, and in that the cross section of the core (4) varies along the circumference of the ring (2, 3, 10), that is to say decreases in the direction of that portion (4b, 10a) which is to be more flexible, in such a way as to permit a deformation of the ring (2, 3, 10) in all planes.

2. Prosthetic ring according to Claim 1, characterized in that the cross section of the core decreases in at least one transverse direction, in particular along the thickness and/or the height of the core (4, 10), symmetrically or asymmetrically in relation to its centre.

3. Prosthetic ring according to Claim 1 or Claim 2, characterized in that the reduction in the cross section of the core (4, 10) is gradual, either regular or irregular.

4. Prosthetic ring according to one of Claims 1 to 3, characterized in that the core (4) is formed by molding a synthetic material.

5. Prosthetic ring according to one of Claims 1 to 3, characterized in that the core (4) is made of titanium alloy known under the designation TA6V.

6. Prosthetic ring according to either of Claims 4 and 5, characterized in that the flexible part (4b) of the core (4) is covered by an insert of elastomeric material reinforcing it.

7. Prosthetic ring according to one of Claims 1 to 6, characterized in that it comprises a radiopaque element.

8. Prosthetic ring for mitral annuloplasty, according to one of Claims 1 to 7, characterized in that it has a general form substantially of a D shape, in that the cross section of the core (4) varies progressively along the circumference of the ring (2), from its most rigid portion (4a) and in the direction of its most flexible portion (4b), down to a minimum which is situated substantially opposite the median part of the most rigid portion (4a).

9. Prosthetic ring for tricuspid annuloplasty, according to one of Claims 1 to 7, characterized in that it has a general form which is more or less ovoid, and in that the cross section of the core (4) varies progressively along the circumference of the ring (2), from its most rigid portion (4a) and in the direction of its most flexible portion (4b), down to a minimum which is situated substantially opposite the median part of the most rigid portion (4a).

10. Prosthetic ring for aortic annuloplasty, according to one of Claims 1 to 7, characterized in that it has three curved portions (10a) which are connected to one another by commissures (10b) which are more or less perpendicular to the plane of the ring, these commissures (10b) being continued via bearing fingers (10c) allowing it to be secured to the corresponding zone of the aortic ring and of the aortic wall, and in that the cross section of the core changes along the ring (10), within each curved portion (10a), being minimum in the median part of each of these curved portions (10a) and increasing in the direction of the commissures (10b).

## Patentansprüche

1. Ringprothese für mitrale, trikuspidale oder Aorta-Annuloplastik, beinhaltend eine Seele (4), die in einer textilen Hülle (5) angeordnet ist, die ein Mittel für die Naht bildet, wobei diese Seele mindestens einen relativ starren Abschnitt (4a, 10b) und einen demgegenüber relativ flexiblen Abschnitt (4b, 10a) aufweist, **dadurch gekennzeichnet, daß** die Seele (4) einen kompletten Ring (2, 3, 10) bildet, daß sie eine monolitische Struktur hat, das heißt aus einem Stück besteht, und daß der Querschnitt der Seele (4) längs des Umfanges des Ringes (2, 3, 10) variiert, daß heißt sich in Richtung seines Abschnittes (4b, 10a), der flexibler sein muß, verringert, derart, daß ein Deformieren des Ringes (2, 3, 10) in allen Ebenen ermöglicht ist.

2. Ringprothese nach Anspruch 1, **dadurch gekennzeichnet, daß** der Querschnitt der Seele sich in mindestens einer Querrichtung verringert, insbesondere in Richtung der Dicke und/oder der Höhe der Seele (4, 10), und zwar bezüglich seines Zentrums symmetrisch oder asymmetrisch.

3. Ringprothese nach Anspruch 1 oder 2, **dadurch gekennzeichnet, daß** die Verringerung des Querschnittes der Seele (4, 10) progressiv, und zwar gleichmäßig oder nicht, ist.

4. Ringprothese nach einem der Ansprüche 1 bis 3, **dadurch gekennzeichnet, daß** die Seele (4) durch Formguß eines Kunststoffes gebildet ist.

5. Ringprothese nach einem der Ansprüche 1 bis 3, **dadurch gekennzeichnet, daß** die Seele (4) aus einer Titanlegierung gebildet ist, die unter der Referenzbezeichnung TA6V bekannt ist.

6. Ringprothese nach einem der Ansprüche 4 oder 5, **dadurch gekennzeichnet, daß** der flexible Abschnitt (4b) der Seele (4) durch ein Einsatzteil aus elastomerem Material umkleidet ist, das sie verstärkt.

7. Ringprothese nach einem der Ansprüche 1 bis 6, **dadurch gekennzeichnet, daß** sie ein radio-opakes Element aufweist.

8. Ringprothese für die mitrale Annuloplastik, nach einem der Ansprüche 1 bis 7, **dadurch gekennzeichnet, daß** sie eine im wesentlichen D-förmige Grundform aufweist, daß der Querschnitt der Seele (4) progressiv längs des Umfanges des Ringes (2) variiert, ausgehend von seinem starreren Abschnitt (4a) und in Richtung seines flexibleren Abschnittes (4b), bis zu einem Minimum das im wesentlichen gegenüber dem mittleren Bereich des starreren Abschnittes (4a) liegt.

9. Ringprothese für die trikuspidale Annuloplastik, nach einem der Ansprüche 1 bis 7, **dadurch gekennzeichnet, daß** sie eine mehr oder weniger ovale Grundform aufweist, und daß der Querschnitt der Seele (4) progressiv längs des Umfanges des Ringes (2) variiert, ausgehend von seinem starreren Abschnitt (4a) und in Richtung seines flexibleren Abschnittes (4b), bis zu einem Minimum, das im wesentlichen dem mittleren Bereich des starreren Abschnittes (4a) gegenüberliegt.

10. Ringprothese für die Aorta-Annuloplastik, nach einem der Ansprüche 1 bis 7, **dadurch gekennzeichnet, daß** sie drei bogenförmige Abschnitte (10a) aufweist, die miteinander durch zur Ebene des Ringes mehr oder weniger senkrechte Verspleißungen (10b) verbunden sind, wobei diese Verspleißungen (10b) durch Stützfinger (10c) verlängert sind, die die Befestigung auf der korrespondierenden Zone des Aorta-Ringes und der Aorta-Wand ermöglichen, und daß der Querschnitt der Seele sich längs des Ringes (10) innerhalb jedes bogenförmigen Abschnittes (10a) weiterentwickelt, wobei er im mittleren Bereich eines jeden dieser bogenförmigen Abschnitte (10a) sein Minimum hat und sich in Richtung auf die Verspleißungen (10b) vergrößert.
